# EUROPEAN PATENT SPECIFICATION

(11) **EP 4 265 287 B1**
(45) Date of publication and mention of the grant of the patent: **02.04.2025**
(21) Application number: 20965605.7
(22) Date of filing: 18.12.2020
(51) Int. Cl.: A61M 11/00, A61M 15/00, B05B 17/06

(54) **ELECTRONIC ATOMIZATION DEVICE**
ELEKTRONISCHE ZERSTÄUBUNGSVORRICHTUNG
DISPOSITIF D'ATOMISATION ÉLECTRONIQUE

(43) Date of publication of application: 25.10.2023
(73) Proprietor: Shenzhen Smoore Technology Limited, Shenzhen, Guangdong 518102 (CN)
(72) Inventor: ZHANG, Xieen, Shenzhen, Guangdong 518102 (CN); CHENG, Shiyi, Shenzhen, Guangdong 518102 (CN)
(74) Representative: Herrero & Asociados, S.L.
(86) International application number: PCT/CN2020/137571
(87) International publication number: WO 2022/126589

(56) References cited:
- WO-A1-2018/172561
- WO-A1-2020/227717
- CN-A- 106 422 005
- CN-A- 110 420 369
- CN-A- 111 744 081
- CN-B- 107 970 505
- CN-U- 206 261 814
- CN-U- 211 678 341
- JP-A- 2008 168 222
- US-A- 5 435 282
- US-A1- 2011 168 172
- US-A1- 2020 197 631
- US-B2- 10 624 391
- US-B2- 10 779 576

## Description

### TECHNICAL FIELD

The present disclosure relates to the field of atomizer technologies, and specifically, to an electronic atomization device.

### BACKGROUND

In the treatment of respiratory system diseases, an atomization inhalation therapy is an important and effective treatment. The atomization inhalation therapy is to atomize medicinal liquid into tiny droplets by using an atomizer. Patients inhale the medicinal liquid into the respiratory tracts and the lungs through breathing, and the medicinal liquid is deposited in the respiratory tracts or the lungs, so as to achieve the purpose of painless, rapid and effective treatment.

After being used by the patients, the conventional atomizer usually does not stop working until the medicinal liquid in the atomizer is consumed, an atomized dose of the medicinal liquid cannot be precisely controlled, and it is easy for the patients to inhale an excessive dose of the medicinal liquid or an insufficient dose of the medicinal liquid. As a result, an expected treatment effect cannot be achieved.

US2020/197631A1 provides self-puncturing liquid drug cartridges and an associated inhaler used to deliver one or more separate doses of an aerosolized liquid drug. A cartridge includes a needle assembly coupled to a drug container. The needle assembly includes a hollow needle and is reconfigurable from a first configuration to a second configuration upon insertion of the cartridge into the inhaler. In the first configuration, the hollow needle does not extend into the container. In the second configuration, the hollow needle extends into the container. The inhaler includes an aerosol generator that includes a vibratable membrane that aerosolizes liquid drug ejected from the cartridge for inhalation by a patient.

US2011/168172A1 provides an aerosolization system, and the aerosolization system includes a squeezable container having a resilient container body. The container is configured to deliver a unit dosage of a liquid when squeezed a single time. The system also includes an aerosolizer that is constructed of a housing defining a mouthpiece, and an aerosol generator disposed in the housing. The aerosol generator includes a vibratable membrane having a front face and a rear face, and a vibratable element used to vibrate the membrane. Further, the housing includes an opening that is adapted to receive a unit dosage of the liquid from the container. The opening provides a liquid path to the rear face of the vibratable membrane.

US5435282A provides a hand-held inhalation responsive nebuliser by which a nebulised air stream is created for quick and efficient delivery of a fluid medication to the respiratory tract of a patient through the deep lung. The nebuliser includes a normally open electrical switch and a rotatable trigger ring extending laterally across an air flow path through which a supply of non-medicated ambient air is drawn during inhalation by the patient. The ambient air supply drawn through the nebuliser causes the trigger ring to rotate into contact with and close the electrical switch, whereby a piezoelectric disk is energized to vibrate an emitter mesh. A metered volume of fluid medication delivered to the emitter mesh from a pre-filled medication cartridge is accelerated and pulled through the mesh as a fine mist of fluid droplets to form a nebulised medication plume. The nebulised medication plume is mixed in the mouthpiece of the nebuliser with the supply of non-medicated ambient air to create the nebulised air stream.

WO2020/227717A1 provides an ultrasonic droplet delivery device and related methods for delivering precise and repeatable amounts of a substance to a user for respiratory use. The ultrasonic droplet delivery device generally includes a body housing, a mouthpiece having an ejector mechanism, and a fluid cartridge having at least one fluid reservoir. In certain embodiments, the ejector mechanism may include at least one ultrasonic actuator and at least one aperture plate with a plurality of openings formed through its thickness for ejecting droplets. The device may further include at least one differential pressure sensor configured to activate the ejector mechanism upon sensing a pre-determined pressure change within the device to thereby generate the ejected stream of droplets.

JP2008168222A provides an ultrasonic atomization device that can prevent clogging without reducing the amount of atomization when dispensing atomized chemical liquid by ultrasonic vibration. The ultrasonic atomization device includes a liquid medicine holding portion that holds a liquid (e.g., liquid medicine) and an ultrasonic generator having a piezoelectric element that generates ultrasonic waves to make the liquid vibrate. The atomizing plate has a deformation opening forming plate that ejects the atomized liquid medicine. The ultrasonic atomization device further includes a liquid medicine conveying mechanism for conveying the liquid medicine atomized by the deformation opening forming plate, and a measuring unit for measuring the liquid medicine that is kept in the liquid medicine holding portion.

CN107970505B provides a portable insulin nebulization drug delivery device, which is provided with a blood glucose detection device, a blood glucose analysis circuit, an ultrasonic circuit for controlling the dosage of administration, a microporous ultrasonic nebulization tablet, etc. The blood glucose analysis circuit automatically inputs the data after the detection and analysis of the patient's blood sample test strip into the ultrasonic circuit for controlling the dosage of administration, and the ultrasonic circuit for controlling the dosage of administration automatically sets the atomized insulin dosage. When inhaling the medicine, the microporous ultrasonic nebulization tablet is energized to work, and the liquid medicine vibrates out from the micropores of several atomizers on the microporous ultrasonic nebulization tablet, and the drug mist particles are inhaled into the alveoli through the nebula nozzle and enter the blood to absorb and take effect, so as to achieve the purpose of controlling blood sugar.

### SUMMARY

In view of this, the present disclosure provides an electronic atomization device, to resolve the technical problem that an atomized dose of medicinal liquid cannot be precisely controlled in the related art.

To resolve the foregoing technical problem, a first technical solution provided in the present disclosure is to provide an electronic atomization device, and the invention is set out in the appended set of claims.

Beneficial effects of the present disclosure are as follows: Different from the related art, the electronic atomization device in the present disclosure includes a microporous atomizing sheet and a needle tube, where one end of the needle tube is spaced apart from the microporous atomizing sheet, and the other end of the needle tube is configured to be inserted into a liquid storage assembly. Liquid in the liquid storage assembly is conveyed to the microporous atomizing sheet by using the needle tube, to precisely control an atomized dose, which can prevent a patient from inhaling excessive medicinal liquid or insufficient medicinal liquid, thereby achieving an expected therapeutic effect for an atomization inhalation therapy.

### BRIEF DESCRIPTION OF THE DRAWINGS

To describe the technical solutions in the embodiments of the present disclosure more clearly, the following briefly introduces the accompanying drawings required for describing the embodiments. Apparently, the accompanying drawings in the following description show only some embodiments of the present disclosure, and a person of ordinary skill in the art may still derive other accompanying drawings from these accompanying drawings without creative efforts.
FIG. 1 is a schematic structural diagram of an electronic atomization device according to the present disclosure.
FIG. 2 is a schematic cross-sectional view of an atomization assembly according to the present disclosure.
FIG. 3 is a three-dimensional structural diagram of an atomization base in an atomization assembly according to the present disclosure.
FIG. 4 is a three-dimensional structural diagram of a first seal member in an atomization assembly according to the present disclosure.
FIG. 5 is a schematic exploded view of a liquid storage assembly according to the present disclosure.
FIG. 6 is a schematic structural diagram of a first embodiment of a control assembly according to the present disclosure.
FIG. 7 is a schematic structural diagram of a drive member in a first embodiment of a control assembly according to the present disclosure.
FIG. 8 is a schematic structural diagram of a push rod in a first embodiment of a control assembly according to the present disclosure.
FIG. 9 is a schematic cross-sectional view of a push rod in a first embodiment of a control assembly according to the present disclosure.
FIG. 10 is a schematic cross-sectional view of another implementation of a push rod in a first embodiment of a control assembly according to the present disclosure.
FIG. 11 is a schematic structural diagram of a second embodiment of a control assembly according to the present disclosure.
FIG. 12 is a schematic cross-sectional view of a second embodiment of a control assembly according to the present disclosure.
FIG. 13 is a schematic exploded view of a second embodiment of a control assembly according to the present disclosure.

### DETAILED DESCRIPTION

The present disclosure is further described in detail below with reference to the accompanying drawings and embodiments. It should be specifically noted that, the following embodiments are merely used for describing the present disclosure rather than limiting the scope of the present disclosure. Similarly, the following embodiments are merely some rather than all of the embodiments of the present disclosure, and all other embodiments obtained by a person of ordinary skill in the art without creative efforts shall fall within the protection scope of the present disclosure.

The terms "first", "second", and "third" in the present disclosure are merely intended for a purpose of description, and shall not be understood as indicating or implying an indication or implication of relative importance or implicitly indicating indication of the number of indicated technical features. Therefore, features defining "first", "second", and "third" can explicitly or implicitly include at least one of the features. In description of the present disclosure, "more" means at least two, such as two and three unless it is specifically defined otherwise. All directional indications (for example, up, down, left, right, front, back...) in the embodiments of the present disclosure are only used for explaining relative position relationships, movement situations, or the like between the various components in a specific posture (as shown in the accompanying drawings). If the specific posture changes, the directional indications change accordingly. Terminologies "comprise", "have", and any variations thereof in the embodiments of the present disclosure are intended to indicate non-exclusive inclusion. For example, a process, method, system, product, or device that includes a series of steps or units is not limited to the listed steps or units, but further optionally includes a step or unit that is not listed, or further optionally includes another step or component that is intrinsic to the process, method, product, or device.

"Embodiment" mentioned in the specification means that particular features, structures, or characteristics described with reference to the embodiment may be included in at least one embodiment of the present disclosure. The term appearing at different positions of the specification may not refer to the same embodiment or an independent or alternative embodiment that is mutually exclusive with another embodiment. A person skilled in the art explicitly or implicitly understands that the embodiments described in the specification may be combined with other embodiments.

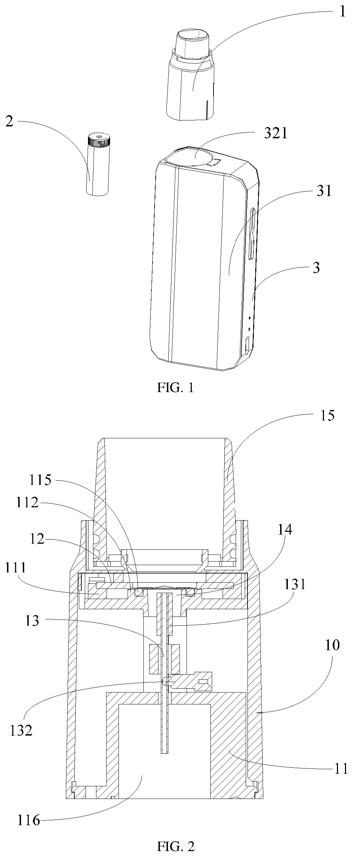

FIG. 1 is a schematic structural diagram of an electronic atomization device according to the present disclosure.

The electronic atomization device may be used to atomize liquid substrates such as medicinal liquid and is applied to medical equipment for treating diseases of upper and lower respiratory, to atomize medical drugs. The electronic atomization device includes an atomization assembly 1, a liquid storage assembly 2, and a control assembly 3. During use, the atomization assembly 1 and the liquid storage assembly 2 are mounted on the control assembly 3. The liquid storage assembly 2 is configured to store medicinal liquid. The atomization assembly 1 is configured to atomize the liquid in the liquid storage assembly 2. The control assembly 3 includes a controller 31 and a mounting cavity 321, the atomization assembly 1 and the liquid storage assembly 2 are mounted in the mounting cavity 321, and the control assembly 3 is configured to convey the liquid in the liquid storage assembly 2 to the atomization assembly 1 and control operation of the atomization assembly 1.

The atomization assembly **1,** the liquid storage assembly 2, and the control assembly 3 may be integrally arranged or detachably connected, which are designed according to specific requirements.

FIG. 2 is a schematic cross-sectional view of an atomization assembly according to the present disclosure.

The atomization assembly 1 includes an atomization shell 10, an atomization base 11, a microporous atomizing sheet 12, and a needle tube 13. The microporous atomizing sheet 12 is arranged on one end of the atomization base 11 and is engaged with an end portion of the atomization base 11 to form an atomization chamber 14. The extending direction of the needle tube 13 is perpendicular to the extending direction of the microporous atomizing sheet 12. Another angle, for example, between 60 degrees and 90 degrees, may alternatively be formed between the extending direction of the needle tube 13 and the extending direction of the microporous atomizing sheet 12, which is designed as required. The needle tube 13 is fixed to the atomization base 11. One end of the needle tube 13 is arranged in the atomization chamber 14 and is spaced apart from the microporous atomizing sheet 12. During use, the other end of the needle tube 13 is inserted into the liquid storage assembly 2, to convey the liquid in the liquid storage assembly 2 to the microporous atomizing sheet 12 to form to-be-atomized liquid. The microporous atomizing sheet 12 is configured to atomize the to-be-atomized liquid. The to-be-atomized liquid is attached between the microporous atomizing sheet 12 and the needle tube 13 through surface tension. A suction nozzle portion 15 is formed or arranged on one end of the atomization shell 10, and the microporous atomizing sheet 12, the needle tube 13, and the atomization base 11 are arranged in the atomization shell 10 together. The suction nozzle portion 15 is in communication with the atomization chamber 14 enclosed by the microporous atomizing sheet 12 and the atomization base 11, and a user inhales, through the suction nozzle portion 15, medicinal liquid atomized by the microporous atomizing sheet 12.

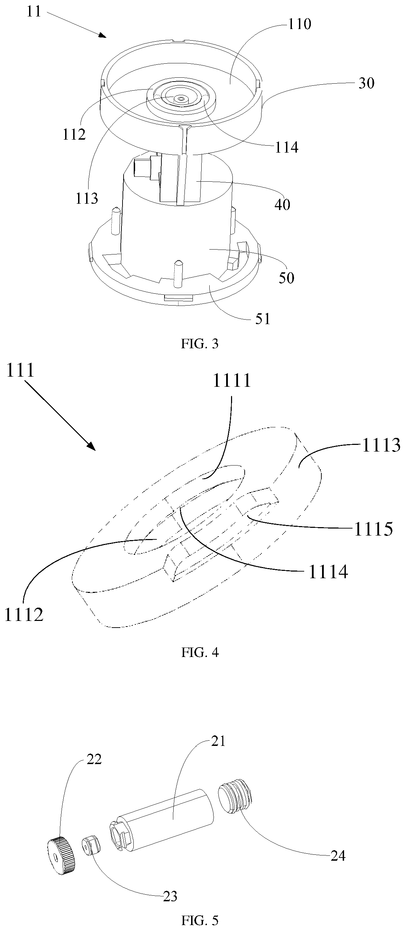

FIG. 3 is a three-dimensional structural diagram of an atomization base in an atomization assembly according to the present disclosure. FIG. 4 is a three-dimensional structural diagram of a first seal member in an atomization assembly according to the present disclosure.

A mounting groove 110 is provided on the end of the atomization base 11, and the mounting groove 110 is configured to mount the microporous atomizing sheet 12. The shape of the mounting groove 110 matches the shape of the microporous atomizing sheet 12. A first seal member 111 is arranged on a periphery of the microporous atomizing sheet 12, and the microporous atomizing sheet 12 and the first seal member 111 are together arranged in the mounting groove 110. The first seal member 111 can fix the microporous atomizing sheet 12, to prevent the microporous atomizing sheet 12 from shaking on the end of the atomization base 11 with affecting operation of the medicinal liquid atomization process.

The microporous atomizing sheet 12 includes a piezoelectric ceramic plate, a metal baseplate, a first electrode, and a second electrode. The first electrode is electrically connected to the piezoelectric ceramic plate, the second electrode is electrically connected to the metal baseplate, and both the first electrode and the second electrode are electrically connected to the controller 31. The metal baseplate is a circular plate, the piezoelectric ceramic plate is a circular ring, and the diameter of the metal baseplate is greater than the inner diameter of the piezoelectric ceramic plate. A through hole is provided in a central region of the piezoelectric ceramic plate, and a region of the metal baseplate corresponding to the central region of the piezoelectric ceramic plate is provided with a plurality of micropores. That is, the microporous atomizing sheet 12 includes a microporous region, the microporous region is provided with a plurality of micropores, and the suction nozzle portion 15 is in communication with the atomization chamber 14 by using the plurality of micropores. In this embodiment, the central region of the metal baseplate protrudes toward the suction nozzle portion 15, to provide a relatively large adhesion surface for the to-be-atomized liquid, thereby increasing adhesion of the to-be-atomized liquid. In another implementation, the metal baseplate may be a planar structure, which is selected as required. This is not limited in the present disclosure.

The first seal member 111 includes a first panel 1111, a second panel 1112, and a side wall 1113. The first panel 1111 is arranged opposite to the second panel 1112. The first panel 1111 is arranged on one end of the side wall 1113, and the second panel 1112 is arranged on the other end of the side wall 1113. That is, the first panel 1111 and the second panel 1112 are arranged at an interval on the side wall 1113, and the side wall 1113 connects the first panel 1111 and the second panel 1112. In an embodiment, the side wall 1113 connects the edge of the first panel 1111 and the edge of the second panel 1112 to form an integral structure. Preferably, the first panel 1111, the second panel 1112, and the side wall 1113 are integrally formed. The first seal member 111 is made of rubber, silicone, or the like.

The first panel 1111 is arranged on a side of the second panel 1112 close to the suction nozzle portion 15. All the first panel 1111, the second panel 1112, and the side wall 1113 are circular ring structures. The outer diameter of the first panel 1111 is the same as the outer diameter of the second panel 1112. The inner diameter of the first panel 1111 may be the same as or different from the inner diameter of the second panel 1112, which is designed as required. The inner diameter of the side wall 1113 is the same as the outer diameter of the first panel 1111 and the outer diameter of the second panel 1112. The thickness of the first panel 1111 is the same as the thickness of the second panel 1112, and a difference between the outer diameter and the inner diameter of the side wall 1113 is the same as the thickness of the first panel 1111 and the thickness of the second panel 1112.

The first panel 1111, the second panel 1112, and the side wall 1113 together enclose an atomizing sheet cavity 1114 for accommodating the microporous atomizing sheet 12. That is, the microporous atomizing sheet 12 is located between the first panel 1111 and the second panel 1112 and is not located beyond the region enclosed by the side wall 1113. A center through hole of the first panel 1111 is in communication with a center through hole of the second panel 1112 and can expose the microporous region on the microporous atomizing sheet 12. In an embodiment, the first panel 1111 and the second panel 1112 are coaxially arranged, and the inner diameter of the first panel 1111 is greater than the inner diameter of the second panel 1112.

An opening 1115 is provided on the first seal member 111, to facilitate mounting of the microporous atomizing sheet 12 into the atomizing sheet cavity 1114. In this embodiment, the opening 1115 is provided at a joint of the side wall 1113 and the first panel 1111, that is, the opening 1115 is formed by cutting off a piece of the edge of the first seal member 111. The opening 1115 may alternatively be provided on the side wall 1113, provided that the microporous atomizing sheet 12 can be mounted in the atomizing sheet cavity 1114, which is not limited in the present disclosure.

In another implementation, the microporous atomizing sheet 12 may alternatively be in another shape such as a square, the structure of the first seal member 111 matches that of the microporous atomizing sheet 12, and the shape of the mounting groove 110 matches that of the microporous atomizing sheet, which is selected as required.

Still referring to FIG. 3, a protrusion 112 is arranged on the bottom wall of the mounting groove 110, and the height of the protrusion 112 is the same as the thickness of the second panel 1112. The protrusion 112 is embedded in the central through hole of the second panel 1112. A hole 113 is provided on the protrusion 112, and the hole 113 corresponds to the microporous region of the microporous atomizing sheet 12. That is, the end of the atomization base 11 engaged with the microporous atomizing sheet 12 is provided with the hole 113, the microporous atomizing sheet 12 covers the hole 113, the microporous region is suspended in the hole 113, and the microporous atomizing sheet 12 is engaged with the hole 113 to form the atomization chamber 14. An annular groove 114 is provided around the hole 113 on the protrusion 112 for mounting a second seal member 115. The size of the annular groove 114 matches the size of the second seal member 115. That is, the end of the atomization base 11 engaged with the microporous atomizing sheet 12 is provided with the annular groove 114, and the second seal member 115 is arranged in the annular groove 114. A non-microporous region of the microporous atomizing sheet 12 covers the annular groove 114. The second seal member 115 is annular, and the second seal member 115 is made of rubber, silicone, or the like. The annular groove 114 is annular. The second seal member 115 is configured to prevent liquid pumped by the needle tube 13 from leaking out of the atomization chamber 14, causing the precision of the atomized dose of the medicinal liquid to be reduced. That is, the annular groove 114 is provided on the protrusion 112, and the second seal member 115 is arranged in the annular groove 114. A projection of the annular groove 114 is on a plane in which the microporous atomizing sheet 12 is located, and the annular groove 114 is arranged around the microporous region of the microporous atomizing sheet 12, that is, the inner diameter of the annular groove 114 is greater than the diameter of the microporous region.

In an embodiment, the cross section of the protrusion 112 is circular, the cross section of the hole 113 is circular, and the protrusion and the opening are concentrically arranged. The outer diameter of the annular groove 114 is equal to the inner diameter of the through hole of the central region of the piezoelectric ceramic plate of the microporous atomizing sheet 12. The second seal member 115 is arranged around the microporous region and abuts against the metal baseplate of the microporous atomizing sheet 12.

During specific implementation, the hole 113 may be a through hole or a blind hole. Specifically, the pore size of the hole 113 is greater than the outer diameter of the needle tube 13, and the end of the needle tube 13 close to the microporous atomizing sheet 12 is spaced apart from the side wall of the hole 113. In this embodiment, the hole 113 is the through hole, and the atomization chamber 14 is an open structure, so that reversely sprayed medicinal liquid can flow out of the atomization chamber 14 along the side wall of the atomization chamber 14, to avoid the impact of the reversely sprayed medicinal liquid on the atomization process. In another implementation, the hole 113 is the blind hole, and the atomization chamber 14 is a closed structure, so that the reversely sprayed medicinal liquid can flow from the side wall of the atomization chamber 14 to the microporous atomizing sheet 12 and finally is deposited on the bottom of the atomization chamber 14, to avoid the impact of the reversely sprayed medicinal liquid on the atomization process. That is, the end of the atomization base 11 engaged with the microporous atomizing sheet 12 is provided with the hole 113, to enable the reversely sprayed medicinal liquid to flow to a direction away from the microporous atomizing sheet 12 along the side wall of the atomization chamber 14 during atomization of the microporous atomizing sheet 12, so as to prevent the reversely sprayed medicinal liquid from forming bubbles or a water film between the needle tube 13 and the microporous atomizing sheet 12 after the medicinal liquid has been atomized, which causes the controller 31 to be unable to accurately detect whether the to-be-atomized liquid still exists and further causes the controller 31 to continue to control the microporous atomizing sheet 12 to perform atomization, resulting in a problem of dry heating, and affecting a service life of the electronic atomization device.

In another implementation, the pore size of the hole 113 is equal to the outer diameter of the needle tube 13, and the micro atomization chamber 14 formed by engaging the microporous atomizing sheet 12 and the end portion of the atomization base 11 is a closed structure and can precisely control the amount of the atomized liquid. It may be understood that the reversely sprayed medicinal liquid cannot flow to a direction away from the microporous atomizing sheet 12 along the side wall of the atomization chamber 14 through the micro atomization chamber 14 of this structure, and the reversely sprayed medicinal liquid affects the atomization process to some extent. In addition, the atomization chamber 14 is the micro atomization chamber 14, and the closed structure also has a probabilistic problem that the liquid is attached to the bottom of the closed structure and cannot be atomized.

Still referring to FIG. 2, the atomization chamber 14, the needle tube 13, and the microporous region of the microporous atomizing sheet 12 are coaxially arranged. The area of a maximum cross section of the atomization chamber 14 is less than four times the area of the microporous region of the microporous atomizing sheet 12. **In** this embodiment, both the cross section of the atomization chamber 14 and the cross section of the microporous region are circular, and the diameter of the atomization chamber 14 is greater than the diameter of the microporous region and is less than twice the diameter of the microporous region. Specifically, the diameter of the atomization chamber 14 ranges from 4 mm to 5 mm, the distance between the end of the needle tube 13 close to the microporous atomizing sheet 12 and the side wall of the atomization chamber 14 ranges from 1.2 mm to 1.8 mm, so that the to-be-atomized liquid pumped by using the needle tube 13 is attached between the microporous atomizing sheet 12 and the needle tube 13, for atomizing the medicinal liquid at any angle, thereby precisely controlling the atomized does of the medicinal liquid. If the diameter of the atomization chamber 14 is excessively large, for example, greater than 5 mm, and the medicinal liquid attached outside the microporous region is increased, an area in which the medicinal liquid is sprayed reversely is increased, the amount of reversely sprayed liquid is increased, and the precision of a dose of the medicinal liquid inhaled by the user is reduced. If the diameter of the atomization chamber 14 is excessively small, for example, less than 4 mm, the to-be-atomized liquid may further flow out to the side wall of the atomization chamber in addition to being attached between the microporous atomizing sheet 12 and the needle tube 13, the residual amount of unatomized medicinal liquid is increased, and the precision of the dose of the medicinal liquid inhaled by the user is reduced.

**In** an embodiment, the atomization chamber 14 is formed by using the hole 113 and the microporous atomizing sheet 12, and the diameter of the atomization chamber 14 is the pore size of the hole 113.

The distance between the end of the needle tube 13 close to the microporous atomizing sheet 12 and the microporous atomizing sheet 12 ranges from 0.2 mm to 0.4 mm. If the distance between the end of the needle tube 13 close to the microporous atomizing sheet 12 and the microporous atomizing sheet 12 is too long, for example, greater than 0.4 mm, the amount of medicinal liquid attached on the side wall of the atomization chamber 14 is increased, a part of the medicinal liquid cannot be attached on the microporous atomizing sheet 12 and cannot be atomized, and the precision of the dose of the medicinal liquid inhaled by the user is reduced. If the distance between the end of the needle tube 13 close to the microporous atomizing sheet 12 and the microporous atomizing sheet 12 is too short, for example, less than 0.2 mm, the medicinal liquid forms bubbles or a water film between the needle tube 13 and the microporous atomizing sheet 12 after the medicinal liquid has been atomized. Consequently, the controller 31 cannot accurately detect whether the to-be-atomized liquid still exists, and the controller 31 continues to control the microporous atomizing sheet 12 to perform atomization, resulting in a problem of dry heating, and affecting a service life of the electronic atomization device.

In an implementation, the end of the needle tube 13 close to the microporous atomizing sheet 12 is provided with a tube cover 131, and the outer wall of the tube cover 131 is spaced apart from the side wall of the atomization chamber 14. The tube cover 131 is configured to increase the surface area of the end of the needle tube 13 close to the microporous atomizing sheet 12, that is, increase a liquid attachment area of the needle tube 13, so as to increase the adhesion of the to-be-atomized liquid. Therefore, the to-be-atomized liquid pumped by the needle tube 13 is better attached between the end of the needle tube 13 close to the microporous atomizing sheet 12 and the microporous atomizing sheet 12. In this embodiment, the tube cover 131 is a hollow cylindrical structure, the inner diameter of the tube cover 131 is the same as the outer diameter of the needle tube 13, and the outer diameter of the tube cover is less than the diameter of the hole 113. The tube cover 131 is made of silicone, rubber, or the like. The tube cover 131 may alternatively be a solid structure, provided that the needle tube 13 is inserted into the tube cover 131.

The needle tube 13 is a hollow metal piece. In this embodiment, the needle tube 13 is a cylindrical metal tube, the inner diameter of the needle tube 13 ranges from 0.7 mm to 1.0 mm, and the needle tube 13 is preferably made of stainless steel. The needle tube 13 may alternatively be a hollow metal piece in another structure, provided that the liquid in the liquid storage assembly 2 can be pumped to the microporous atomizing sheet 12 to form the to-be-atomized liquid. The material of the needle tube 13 only needs to not react with the to-be-atomized medicinal liquid and cause the medicinal liquid to deteriorate.

In an implementation, the needle tube 13 may further be configured for detection. A conductor 132 is arranged on the needle tube 13, and the conductor 132 is electrically connected to the controller 31. **In** this embodiment, a pogo pin is selected for the conductor 132. In another implementation, another element may alternatively be selected for the conductor 132, provided that the needle tube 13 is electrically connected to the controller 31 through the conductor 132.

The metal baseplate in the microporous atomizing sheet 12 is electrically connected to the controller 31 through a wire, the needle tube 13 is electrically connected to the controller 31 through the conductor 132 and a wire, and the needle tube 13 and the metal baseplate form an impedance sensor, that is, the needle tube 13 and the metal baseplate in the microporous atomizing sheet 12 are equivalent to two metal electrodes. After the liquid in the liquid storage assembly 2 is pumped by the needle tube 13, the to-be-atomized liquid is attached between the end of the needle tube 13 close to the microporous atomizing sheet 12 and the microporous atomizing sheet 12, to conduct the metal baseplate in the microporous atomizing sheet 12 and the needle tube 13, and a resistance value between the metal baseplate in the microporous atomizing sheet 12 and the needle tube 13 is small, about 0. After the to-be-atomized liquid is atomized, no to-be-atomized liquid exists between the end of the needle tube 13 close to the microporous atomizing sheet 12 and the microporous atomizing sheet 12, the metal baseplate in the microporous atomizing sheet 12 and the needle tube 13 are in an open circuit state, and a resistance value between the metal baseplate in the microporous atomizing sheet 12 and the needle tube 13 is far greater than 0 and is also greater than the resistance value between the metal baseplate in the microporous atomizing sheet 12 and the needle tube 13 in a conduction state through the to-be-atomized liquid.

Whether the to-be-atomized liquid exists can be determined by detecting the resistance value between the metal baseplate in the microporous atomizing sheet 12 and the needle tube 13 by using the controller 31. That is, if the resistance value between the metal baseplate in the microporous atomizing sheet 12 and the needle tube 13 detected by the controller 31 is close to 0, it is determined that the to-be-atomized liquid exists between the microporous atomizing sheet 12 and the needle tube 13, and the microporous atomizing sheet 12 is controlled to atomize the to-be-atomized liquid. If the resistance value between the metal baseplate in the microporous atomizing sheet 12 and the needle tube 13 detected by the controller 31 is far greater than 0, it is determined that the to-be-atomized liquid between the microporous atomizing sheet 12 and the needle tube 13 is about to be consumed or has been consumed, so that the microporous atomizing sheet 12 is controlled to stop working directly or with a delay (a specific value of the delay is set according to experience, for example, 2s).

In another implementation, the needle tube 13 is made of silicone, plastic, or the like. In this case, the needle tube 13 does not have a detection function and can only be configured to pump the liquid in the liquid storage assembly 2 to the microporous atomizing sheet 12.

Due to the surface tension and the adhesion of the liquid, the liquid is attached on the microporous atomizing sheet 12 after being pumped out from the needle tube 13. The liquid is located between the end of the needle tube 13 close to the microporous atomizing sheet 12 and the microporous atomizing sheet 12 and spreads around to reach the front edge of the atomization chamber 14. During operation, the microporous atomizing sheet 12 changes the liquid into a spray, and the spray is sprayed out from the suction nozzle portion 15. During atomization, with consumption of the liquid, under the action of atmospheric pressure, unatomized liquid continuously moves to the microporous region of the microporous atomizing sheet 12 and is finally atomized completely. Under the comprehensive effect of the surface tension and the adhesion of the liquid and the atmospheric pressure, the liquid conveyed by the needle tube 13 and the liquid atomization process are completely free from the constraints of direction and gravity. Therefore, in another implementation, the needle tube 13 may alternatively be parallel to the microporous atomizing sheet 12, other structures are changed accordingly, and the working principles of the needle tube 13 and the microporous atomizing sheet 12 are the same as the foregoing. Details are not described herein again.

The end of the atomization base 11 away from the microporous atomizing sheet 12 is provided with an accommodating groove 116, and the accommodating groove 116 is configured to accommodate the liquid storage assembly 2. The end of the needle tube 13 away from the microporous atomizing sheet 12 is arranged in the accommodating groove 116, to enable the end of the needle tube 13 away from the microporous atomizing sheet 12 to be inserted into the liquid storage assembly 2, so as to pump the liquid in the liquid storage assembly 2 to the microporous atomizing sheet 12. FIG. 5 is a schematic exploded view of a liquid storage assembly according to the present disclosure.

The liquid storage assembly 2 includes a liquid storage shell 21, a liquid storage cover 22, a seal plug 23, and a piston 24. One end of the liquid storage shell 21 is provided with the liquid storage cover 22, and the other end of the liquid storage shell is provided with the piston 24. One end of the liquid storage cover 22 close to the liquid storage shell 21 is provided with the seal plug 23, and the seal plug 23 is configured to seal the liquid storage shell 21, to prevent the liquid in the liquid storage assembly 2 from leaking. A space enclosed by the liquid storage shell 21, the seal plug 23, and the piston 24 together is a liquid storage tank, and the liquid storage tank is configured to store the to-be-atomized liquid. An opening may be provided on the liquid storage cover 22, to expose a part of the seal plug 23.

The liquid storage assembly 2 is mounted in the mounting cavity 321 of the control assembly 3, and the end of the liquid storage assembly 2 provided with the seal plug 23 faces toward an opening of the mounting cavity 321, to facilitate insertion of the needle tube 13 in the atomization assembly 1 into the liquid storage assembly 2. The end of the liquid storage assembly 2 provided with the piston 24 faces toward the bottom of the mounting cavity 321, so that a component in the control assembly 3 pushes the piston 24, to convey the liquid in the liquid storage assembly 2 to the needle tube 13, so as to reach the microporous atomizing sheet 12.

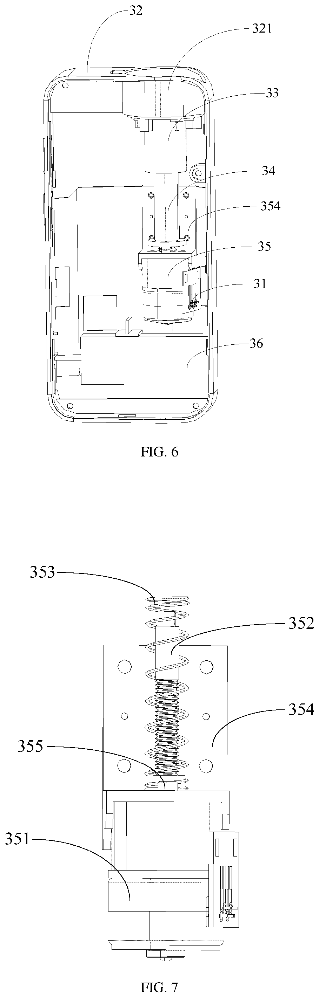

FIG. 6 is a schematic structural diagram of a first embodiment of a control assembly according to the present disclosure. The control assembly 3 further includes a control shell 32, an accommodating base 33, a push rod 34, a drive member 35, and a battery 36.

One end of the control shell 32 is provided with the mounting cavity 321, and the mounting cavity 321 is configured to accommodate the atomization assembly 1 and a part of the liquid storage assembly 2. The part of the liquid storage assembly 2 is arranged in the accommodating groove 116 of the atomization assembly 1 and is accommodated in the mounting cavity 321 together with the atomization assembly 1. The structure of the mounting cavity 321 may be a ring. In this embodiment, the mounting cavity 321 is a circular ring. The mounting cavity 321 and the control shell 32 are fixed together through adhesive, bolts, or the like. Preferably, the mounting cavity 321 and the control shell 32 are integrally formed. **In** an embodiment, the control shell 32 includes a top wall and a bottom wall that are spaced apart from each other and an annular side wall that connects the top wall and the bottom wall. A through hole is provided at a position of the top wall close to the side wall and is used as the mounting cavity 321, and the internal space of the control shell 32 is in communication with the outside through the through hole.

The accommodating base 33 is arranged in the control shell 32 and is fixedly connected to the control shell 32.

The accommodating base 33 is arranged on one end of the mounting cavity 321 close to the bottom wall of the control shell 32, and the internal space of the accommodating base 33 is in communication with the mounting cavity 321. The accommodating base 33 and the mounting cavity 321 may be integrally formed. The accommodating base 33 is configured to accommodate the part of the liquid storage assembly 2. After the atomization assembly 1 is inserted into the mounting cavity 321, one end of the accommodating base 33 close to the mounting cavity 321 is fixedly connected to the atomization base 11 in the atomization assembly 1 in a manner such as bolts, fastening, or magnetic member adsorption. In this embodiment, the end of the accommodating base close to the mounting cavity and the atomization base are fixed through bolts. Both the end of the accommodating base 33 close to the mounting cavity 321 and one end of the atomization base 11 close to the accommodating base 33 are provided with mounting structures (for example, mounting holes), to fix the atomization base 11 and the accommodating base 33 together.

The push rod 34 is arranged on one end of the accommodating base 33 away from the mounting cavity 321. The push rod 34 is movably connected to the accommodating base 33, and the push rod 34 abuts against the liquid storage assembly 2 arranged in the accommodating base 33.

One end of the push rod 34 is partially accommodated in the accommodating base 33, and one end of the push rod 34 close to the drive member 35 and the drive member 35 are located outside the accommodating base.

The drive member 35 is arranged on the end of the push rod 34 away from the accommodating base 33. The drive member 35 is configured to drive the push rod 34 to move toward the liquid storage assembly 2, to enable the push rod 34 to push the piston 24 in the liquid storage assembly 2 to move toward the atomization assembly 1, so as to convey the liquid in the liquid storage assembly 2 to the microporous atomizing sheet 12.

The battery 36 is configured to provided electric energy for operation of the microporous atomizing sheet 12 and the drive member 35. The controller 31 is configured to control working statuses of the microporous atomizing sheet 12 and the drive member 35, that is, the controller 31 controls whether the battery 36 supplies power to the microporous atomizing sheet 12 and the drive member 35. After the controller 31 controls the drive member 35 to start, the drive member 35 drives the push rod 34 to move toward the accommodating base 33, so as to convey a predetermined amount of medicinal liquid in the liquid storage assembly 2 to the atomization chamber 14 through the needle tube 13. After detecting that to-be-atomized medicinal liquid exists between the needle tube 13 and the microporous atomizing sheet 12 in the atomization chamber 14, the controller 31 controls the microporous atomizing sheet 12 to perform an atomization operation. After detecting that the medicinal liquid between the needle tube 13 and the microporous atomizing sheet 12 in the atomization chamber 14 has been atomized, the controller 31 controls the microporous atomizing sheet 12 to stop working. Because each moving distance of the push rod 34 may be controlled, the predetermined amount of medicinal liquid may further be controlled to be conveyed to the atomization chamber 14 for atomization, to precisely control the amount of atomized liquid.

FIG. 7 is a schematic structural diagram of a drive member in a first embodiment of a control assembly according to the present disclosure.

The drive member 35 includes a motor 351 and a screw rod 352 rotatably connected to the motor 351.

The motor 351 is fixed to the side wall of the control shell 32 by using a support element 354, and the screw rod 352 is arranged on one end of the motor 351 close to the push rod 34. That is, the motor 351 is spaced apart from the accommodating base 33. The end of the motor 351 close to the push rod 34 is provided with a first contact element 355, and the first contact element 355 is electrically connected to the controller 31. The material of the first contact element 355 may be, but not limited to, metal, provided that the first contact element can perform conduction. In this embodiment, the first contact element 355 is cylindrical.

In another implementation, the first contact element 355 may be sheet-shaped or another structure, which is designed as required.

An elastic element 353 is sleeved on the screw rod 352. In this embodiment, the elastic element 353 is a spring. In another implementation, the elastic element 353 may alternatively be another element that can be deformed and can be restored to an original state, provided that the element can meet the requirements.

In another implementation, the drive member 35 may include the motor 351 and a gear rotatably connected to the motor 351, and a tooth matching the push rod is arranged on the corresponding push rod 34, so that the drive member 35 drives the push rod 34 to move. Provided that the drive member 35 can drive the push rod 34 to move in an extending direction of the push rod. The specific structures of the drive member 35 and the push rod 34 may be designed according to requirements.

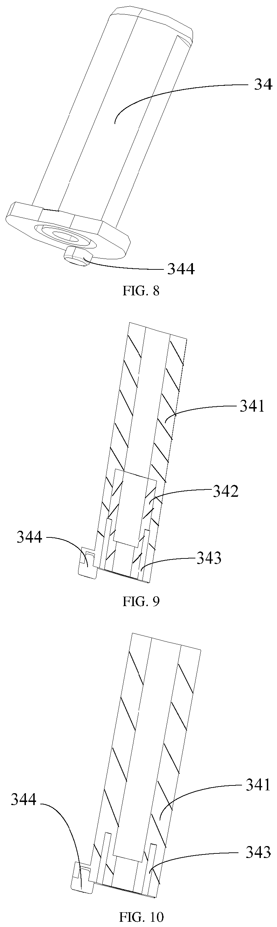

FIG. 8 is a schematic structural diagram of a push rod in a first embodiment of a control assembly according to the present disclosure. FIG. 9 is a schematic cross-sectional view of a push rod in a first embodiment of a control assembly according to the present disclosure. FIG. 10 is a schematic cross-sectional view of another implementation of a push rod in a first embodiment of a control assembly according to the present disclosure.

The end of the push rod 34 away from the accommodating base 33 is provided with a thread. The end of the push rod 34 away from the accommodating base 33 is sleeved on the screw rod 352, that is, the end of the push rod 34 provided with the thread is rotatably connected to the screw rod 352. The thread on the push rod 34 matches a thread on the screw rod 352. When the screw rod 352 rotates, the thread on the push rod 34 moves up and down along the screw rod 352. Under the drive of the screw rod 352, the push rod 34 moves toward the accommodating base 33, to push the piston 24 in the liquid storage assembly 2 to move to squeeze the medicinal liquid. A quantity of rotation circles of the screw rod 352 in a single time is controlled, so that strokes of the push rod 34 and the piston 24 are controlled, to finally achieve accurate liquid feeding.

To precisely control the amount of liquid pumped out from the liquid in the liquid storage assembly 2 by using the needle tube 13, the precision of the thread on the screw rod 352 and the precision of the thread on the push rod 34 are set to be less than or equal to level 5, to improve the precision of a single rotation and to precisely control the moving distance of the push rod 34, thereby precisely controlling the atomized dose. It may be understood that the selection of the thread precision is also associated with a requirement of atomization precision, higher precision indicates higher atomization precision, and a smaller value set for the thread precision indicates higher precision.

**In** this embodiment, as shown in FIG. 9, the push rod 34 includes a push rod body 341 and a nut 342, and the nut 342 is arranged on the end of the push rod 34 away from the accommodating base 33 and is arranged on the inner wall of the push rod body 341. **In** another implementation, as shown in FIG. 10, the push rod 34 includes the push rod body 341 and a thread formed on the inner wall of the push rod body 341, and the thread is arranged on the end of the push rod body 341 away from the accommodating base 33.

One end of the push rod body 341 close to the drive member 35 is provided with a limiting groove 343, and the limiting groove 343 is provided around the thread on the push rod body 341. The limiting groove 343 is configured to accommodate the elastic element 353 sleeved on the screw rod 352. When the push rod 34 is sleeved on the screw rod 352, one end of the elastic element 353 abuts against the bottom wall of the limiting groove 343, and the other end of the elastic element abuts against the motor 351. With the rotation of the screw rod 352, the elastic element 353 is compressed, the elastic element 353 applies a force that is opposite to a moving direction of the push rod to the push rod 34, to eliminate a gap between the thread on the screw rod 352 and the thread on the push rod 34, so that the thread on the screw rod 352 matches the thread on the push rod 34 more closely, to precisely control the moving distance of the push rod 34. In another implementation, the elastic element 353 is fixedly connected to the motor 351, to eliminate the gap between the thread on the screw rod 352 and the thread on the push rod 34.

The end of the push rod 34 close to the drive member 35 is provided with a second contact element 344, and the second contact element 344 is electrically connected to the controller 31. A material of the second contact element 344 may be, but not limited to, metal, provided that the second contact element can perform conduction. In this embodiment, the second contact element 344 is cylindrical. A sum of the height after the first contact element 355 abuts against the second contact element 344 and the depth of the limiting groove 343 is the same as the height of the elastic element 353 after being compressed to the greatest extent. In another implementation, the second contact element 344 may be sheet-shaped or another structure, which may be designed as required.

When the screw rod 352 drives the push rod 34 to move away from the accommodating base 33, that is, drives the push rod 34 to move toward the motor 351, and after the first contact element 355 is in contact with the second contact element 344, the controller 31 detects that the first contact element 355 is conducted to the second contact element 344 and controls the motor 351 to stop rotating, so that the push rod 34 stops moving toward the motor 351, to restrict a downward movement position of the push rod 34.

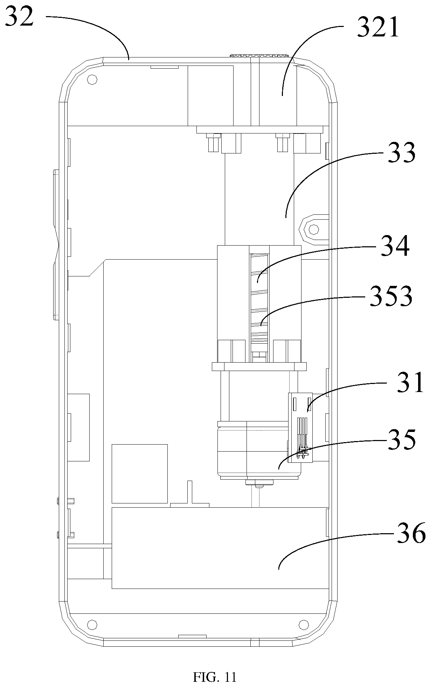

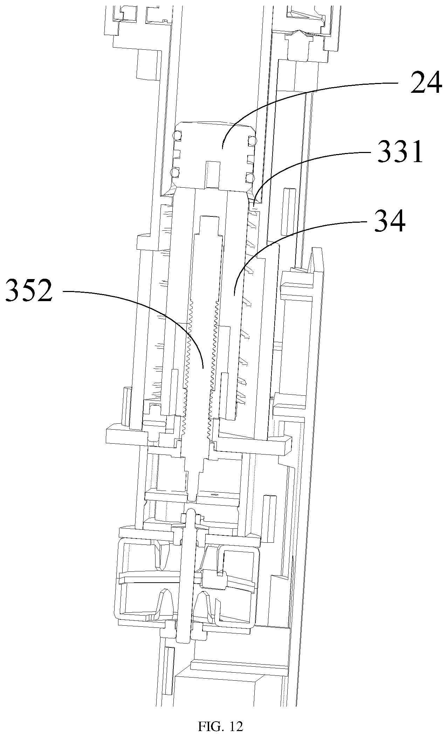

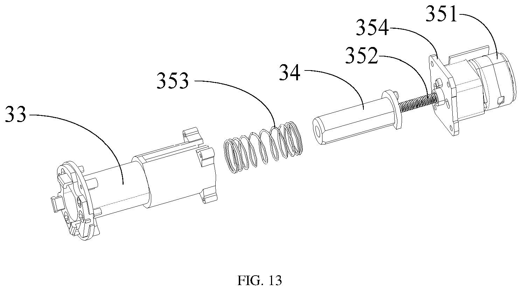

FIG. 11 is a schematic structural diagram of a second embodiment of a control assembly according to the present disclosure. FIG. 12 is a schematic cross-sectional view of a second embodiment of a control assembly according to the present disclosure. FIG. 13 is a schematic exploded view of a second embodiment of a control assembly according to the present disclosure.

In the second embodiment, the structure of the control assembly 3 is substantially the same as that in the first embodiment, except that the structure of the accommodating base 33, the arrangement position of the elastic element 353, and the connection relationship between the accommodating base 33 and the push rod 34 as well as the drive member 35.

In this embodiment, the accommodating base 33, the push rod 34, and the drive member 35 are of an integral structure. One end of the accommodating base 33 is fixed to the mounting cavity 321 and is in communication with the mounting cavity 321. The other end of the accommodating base 33 is fixed to the support element 354. A part of the liquid storage assembly 2 is accommodated in the accommodating base 33. The push rod 34 and the screw rod 352 of the drive member 35 are arranged in the accommodating base 33 as a whole. The motor 351 is arranged on a side of the support element 354 away from the accommodating base 33, and the accommodating base 33 is fixedly connected to the motor 351 by the support element 354. It may be understood that to improve the precision of the atomized dose, the rigidity of the entire structure, that is, the ability not to deform, needs to be improved, that is, it is expected that the position of the push rod 34 does not change due to another factor during rotation of the screw rod 352. The accommodating base 33, the push rod 34, and the drive member 35 are set to an integral structure, which can prevent the support element 354 of the drive member 35 from being slightly deformed in the moving direction of the push rod 34 when the screw rod 352 drives the push rod 34 to move, causing the thrust of the motor 351 to be offset. The accommodating base 33 is fixedly connected to the support element 354, and the accommodating base 33 is fixed to the control shell 32, to prevent the support element 354 from being deformed, thereby improving the precision of the atomized dose.

A limiting element 331 is arranged in the accommodating base 33, and the limiting element 331 is an annular structure and is arranged on the inner wall of the accommodating base 33. The limiting element 331 may be fixedly connected to the accommodating base 33 through adhesive or the like. Preferably, the limiting element 331 and the accommodating base 33 are integrally formed. The limiting element 331 is arranged on one end of the liquid storage assembly 2 close to the drive member 35 and abuts against the liquid storage assembly 2. That is, the limiting element 331 is arranged on the end of the push rod 34 close to the liquid storage assembly 2.

The limiting element 331 is configured to prevent the push rod 34 from rotating with the rotation of the screw rod 352, that is, to limit shaking of the push rod 34. A gap between the push rod 34 and the limiting element 331 is controlled within 0.05 mm, to prevent the push rod 34 from shaking to the greatest extent, and to precisely control the moving distance of the push rod 34, thereby precisely controlling the atomized dose.

The elastic element 353 is sleeved on the push rod 34, that is, the push rod 34 is elastically connected to the accommodating base 33.

In this embodiment, the elastic element 353 is a spring, one end of the spring abuts against the limiting element 331, and the other end of the spring is fixed to a flange of the outer wall of one end of the push rod 34 close to the motor 351. With the rotation of the screw rod 352, the push rod 34 moves toward the piston 24, the elastic element 353 is compressed, the elastic element 353 applies a force that is opposite to a moving direction of the push rod to the push rod 34, to eliminate a gap between the thread on the screw rod 352 and the thread on the push rod 34, so that the thread on the screw rod 352 matches the thread on the push rod 34 more closely, and the push rod 34 does not shake, to precisely control the moving distance of the push rod 34. In another implementation, the elastic element 353 may alternatively be another element that can be deformed and can be restored to an original state, provided that the element meets the requirements.

The electronic atomization device in the present disclosure includes a microporous atomizing sheet and a needle tube, where one end of the needle tube is spaced apart from the microporous atomizing sheet, and the other end of the needle tube is configured to be inserted into a liquid storage assembly; and the needle tube is configured to convey liquid in the liquid storage assembly to the microporous atomizing sheet to form to-be-atomized liquid; and the microporous atomizing sheet is configured to atomize the to-be-atomized liquid, and the to-be-atomized liquid is attached between the microporous atomizing sheet and the needle tube through surface tension. Liquid in the liquid storage assembly is conveyed to the microporous atomizing sheet by using the needle tube, to precisely control the atomized dose, which can prevent a patient from inhaling excessive medicinal liquid or insufficient medicinal liquid, thereby achieving an expected therapeutic effect for an atomization inhalation therapy.

The foregoing descriptions are merely some embodiments of the present disclosure, and the protection scope of the present disclosure is not limited thereto. All equivalent apparatus or process changes made according to the content of this specification and accompanying drawings in the present disclosure or by directly or indirectly applying the present disclosure in other related technical fields shall fall within the protection scope of the present disclosure. The embodiments being not covered by the independent claim do not form part of the claimed invention, but represent only background art or examples that are useful for understanding the invention.

## Claims

1. An electronic atomization device, comprising:
a microporous atomizing sheet (12);
a needle tube (13), wherein one end of the needle tube (13) is spaced apart from the microporous atomizing sheet (12), and the other end of the needle tube (13) is configured to be inserted into a liquid storage assembly (2); the needle tube is configured to convey liquid in the liquid storage assembly to the microporous atomizing sheet to form to-be-atomized liquid; and the to-be-atomized liquid is attached between the microporous atomizing sheet and the needle tube through surface tension; and
an atomization base (11), wherein the microporous atomizing sheet (12) is arranged on one end of the atomization base and is engaged with an end portion of the atomization base (11) to form an atomization chamber (14); and the needle tube (13) is fixed to the atomization base (11), and the end of the needle tube (13) is arranged in the atomization chamber (14) and is spaced apart from the microporous atomizing sheet;
wherein the microporous atomizing sheet (12) comprises a microporous region, both the cross section of the atomization chamber (14) and the cross section of the microporous region are circular; and the atomization chamber (14), the microporous region of the microporous atomizing sheet (12), and the needle tube (13) are coaxially arranged;
**characterized in that** the diameter of the atomization chamber (14) is greater than the diameter of the microporous region and is less than twice the diameter of the microporous region.

2. The electronic atomization device of claim 1, wherein the diameter of the atomization chamber (14) ranges from 4 mm to 5 mm; and the distance between the end of the needle tube (13) close to the microporous atomizing sheet (12) and the side wall of the atomization chamber (14) ranges from 1.2 mm to 1.8 mm.

3. The electronic atomization device of claim 1, wherein the distance between the end of the needle tube (13) close to the microporous atomizing sheet (12) and the microporous atomizing sheet (12) ranges from 0.2 mm to 0.4 mm.

4. The electronic atomization device of claim 1, wherein the end of the atomization base (11) engaged with the microporous atomizing sheet (12) is provided with an annular groove (114), and a seal member (115) is arranged in the annular groove (114); and a non-microporous region of the microporous atomizing sheet (12) covers the annular groove (114).

5. The electronic atomization device of claim 4, wherein the annular groove (114) is annular, the seal member is annular, and the seal member (115) is made of silicone.

6. The electronic atomization device of claim 1, wherein the end of the atomization base (11) engaged with the microporous atomizing sheet (12) is provided with a hole (113), the microporous atomizing sheet (12) covers the hole (113) and is engaged with the hole (113) to form the atomization chamber (14), and the end of the needle tube (13) close to the microporous atomizing sheet (12) is spaced apart from the side wall of the hole (113).

7. The electronic atomization device of claim 6, wherein the hole (113) is a through hole or a blind hole.

8. The electronic atomization device of claim 1, wherein the end of the needle tube (13) close to the microporous atomizing sheet (12) is provided with a tube cover (131), and the tube cover is configured to increase adhesion of the to-be-atomized liquid.

9. The electronic atomization device of claim 8, wherein the outer wall of the tube cover (131) is spaced apart from the side wall of the atomization chamber (14), and the tube cover (131) is made of silicone.

10. The electronic atomization device of claim 1, wherein the needle tube (13) is a cylindrical metal tube, and the inner diameter of the needle tube (13) ranges from 0.7 mm to 1.0 mm.

11. The electronic atomization device of claim 10, further comprising a controller (31), wherein a conductor is arranged on the needle tube (13), and the conductor is electrically connected to the controller (31); the microporous atomizing sheet (12) is electrically connected to the controller (31), and the needle tube (13) and the microporous atomizing sheet (12) form an impedance sensor; and the controller (31) detects the resistance value between the needle tube (13) and the microporous atomizing sheet (12) and controls an operating state of the microporous atomizing sheet (12) according to the resistance value.

12. The electronic atomization device of claim 1, wherein the end portion of the atomization base (11) away from the microporous atomizing sheet (12) is provided with an accommodating groove (116), the accommodating groove is configured to accommodate the liquid storage assembly, and the end of the needle tube (13) away from the microporous atomizing sheet (12) is arranged in the accommodating groove (116).

## Patentansprüche

1. Eine elektronische Zerstäubungsvorrichtung, die Folgendes umfasst:
Eine mikroporöse Zerstäubungsfolie (12);
eine Nadelröhre (13), wobei ein Ende der Nadelröhre mit bezug auf die poröse Zerstäubungsfolie (12) einen Abstand aufweist und das andere Ende der Nadelröhre (13) konfiguriert ist, um in eine Baugruppe (2) für die Flüssigkeitsspeicherung eingefügt zu werden; wobei die Nadelröhre konfiguriert ist, um Flüssigkeit in die Baugruppe (2) für die Flüssikeitspeicherung bis zur mikroporösen Zerstäubungsfolie zu fördern, um die zu zerstäubende Flüssigkeit zu bilden; und wobei die zu zerstäubende Flüssigkeit zwischen der mikroporösen Zerstäubungsfolie und der Nadelröhre über die Oberflächenspannung zurückgehalten wird; und
eine Zerstäubungsgrundfläche (11), wobei die mikroporöse Zerstäubungsfolie (12) an einem Ende der Zerstäubungsgrundfläche angeordnet und in ein Endteil der Zerstäubungsgrundfläche eingreift, um eine Zerstäubungskammer (14) zu bilden, und wobei die Nadelröhre (13) an der Zerstäubungsgrundfläche (11) befestigt ist und das Ende der Nadelröhre (13) in der Zerstäubungskammer (14) angeordnet ist und mit bezug auf die mikorporösen Zerstäubungsfolie einen Abstand aufweist;
wobei die mikorporöse Zerstäubungfolie (12) einen mikroporösen Bereich umfasst, sowohl der Querschnitt der Zerstäubungskammer (14) als auch der Querschnitt des mikorporösen Bereiches kreisförmig sind; und die Zerstäubungskammer (14), der mikroporöse Bereich der mikroporösen Zerstäubungsfolie (12) und die Nadelröhre (13) koaxial angeordnet sind;
**dadurch gekennzeichnet, dass** der Durchmesser der Zerstäubungskammer (14) grösser ist als der Durchmesser des mikroporösen Bereiches und kleiner als zweimal der Durchmesser des mikroporösen Bereiches.

2. Die elektronische Zerstäubungsvorrichtung gemäss Anspruch 1, bei der der Durchmesser der Zerstäubungskammer (14) zwischen 4 mm und 5 mm liegt; und der Abstand zwischen dem Ende der Nadelröhre (13) nahe der mikroporösen Zerstäubungsfolie (12) und der Seitenwand der Zerstäubungskammer zwischen 1,2 mm und 1,8 mm beträgt.

3. Die elektronische Zerstäubungsvorrichtung gemäss Anspruch 1, bei der der Abstand zwischen dem Ende der Nadelröhre (13) nahe der mikroporösen Zerstäubungsfolie (12) und der mikroporösen Zerstäubungsfolie (12) 0,2 mm bis 0,4 mm beträgt.

4. Die elektronische Zerstäubungsvorrichtung gemäss Anspruch 1, bei der das Ende der Zerstäubungsgrundfläche (11), die in die mikroporöse Zerstäubungsfolie (12) eingreift, mit einer ringförmigen Rille (114) versehen ist, und ein Dichtungsglied (115) in der ringförmigen Rille (114) angeordnet ist; und ein nicht mikroporöser Bereich der mikroporösen Zerstäubungsfolie (12) die ringförmige Rille (114) deckt.

5. Die elektronische Zerstäubungsvorrichtung gemäss Anspruch 4, bei der die ringförmige Rille (114) ringförmig, das Dichtungsglied ringförmig und das Dichtungsglied (115) aus Silikon ist.

6. Die elektronische Zerstäubungsvorrichtung gemäss Anspruch 1, bei der das Ende der Zerstäubungsgrundfläche (11), das in die mikroporöse Zerstäubungsfolie (12) eingreift, mit einem Loch (113) versehen ist, die mikroporöse Zerstäubungsfolie (12) das Loch (113) deckt und in das Loch (113) eingreift, um eine Zerstäubungskammer (14) zu bilden, und wobei das Ende der Nadelröhre (13) nahe der mikroporösen Folie (12) mit bezug auf die Seitenwand des Loches (113) einen Abstand aufweist..

7. Die elektronische Zerstäubungsvorrichtung gemäss Anspruch 6, bei der das Loch (113) ein Durchgangsloch oder ein Sackloch ist.

8. Die elektronische Zerstäubungsvorrichtung gemäss Anspruch 1, bei der das Ende der Nadelröhre (13) nahe der mikroporösen Zerstäubungsfolie (12) mit einer Röhrenhülle (131) bedeckt ist, und die Röhrenhülle konfiguriert ist, um die Haftung der zu zerstäubenden Flüssigkeit zu steigern.

9. Die elektronische Zerstäubungsvorrichtung gemäss Anspruch 8, bei der die Aussenwand der Röhrenhülle (131) mit bezug auf die Seitenwand der Zerstäubungskammer (14) einen Abstand aufweist und die Röhrenhülle (131) aus Silikon gefertigt ist.

10. Die elektronische Zerstäubungsvorrichtung gemäss Anspruch 1, bei der die Nadelröhre (13) eine zylinderförmige Metallröhre ist, und der Innendurchmesser der Nadelröhre (13) 0,7 mm bis zu 1,0 mm misst.

11. Die elektronische Zerstäubungsvorrichtung gemäss Anspruch 10, die weiter ein Steuergerät (31) umfasst, wobei an der Nadelröhre (13) ein Leiter angeordnet ist, und der Leiter elektrisch mit dem Steuergerät (31) verbunden ist; wobei die mikroporöse Zerstäubungsfolie (12) elektrisch mit dem Steuergerät (31) verbunden ist, und die Nadelröhre (13) und die mikorporöse Zerstäubungsfolie (12) einen Impedanzsensor bilden; und wobei das Steuergerät (31) den Widerstandswert zwischen der Nadelröhre (13) und der mikroporösen Zerstäubungsfolie (12) ermittelt und einen Betriebszustand der mikroporösen Zerstäubungsfolie (12) übereinstimmend mit dem Widerstandswert steuert.

12. Die elektronische Zerstäubungsvorrichtung gemäss Anspruch 1, bei der der Endteil der Zerstäubungsgrundfläche (11), das weggerichtet ist von der mikroporösen Zerstäubungsfolie (12), mit einer Aufnahmerille (116) versehen ist, wobei die Aufnahmerille zur Aufnahme der Baugruppe für die Flüssigkeitsspeicherung konfiguriert ist, und wobei das von der mikroporösen Zerstäubungsfolie (12) abgewandte Ende der Nadelröhre (13) in der Aufnahmerille (116) angeordnet ist.

## Revendications

1. Un dispositif électronique d'atomisation, comprenant :
une feuille d'atomisation microporeuse (12) ;
un tube à aiguille (13), dans lequel une extrémité du tube à aiguille (13) est éloignée de la feuille d'atomisation microporeuse (12), et l'autre extrémité du tube à aiguille (13) est configurée pour être insérée dans un ensemble de stockage de liquide (2) ; dans lequel le tube à aiguille est configuré pour transporter le liquide contenu dans l'ensemble de stockage de liquide vers la feuille d'atomisation microporeuse pour former un liquide à atomiser : et dans lequel le liquide à atomiser est fixé entre la feuille d'atomisation microporeuse et le tube à aiguille par la tension superficielle ; et
une base d'atomisation (11), dans laquelle la feuille d'atomisation microporeuse (12) est disposée à une extrémité de la base d'atomisation et est engagée avec une portion d'extrémité de la base d'atomisation (11) pour former une chambre d'atomisation (14) ; et dans laquelle le tube à aiguille (13) est fixé à la base d'atomisation (11),
et l'extrémité du tube à aiguille (13) est disposée dans la chambre d'atomisation (14) et est séparée par rapport à la feuille d'atomisation microporeuse ;
dans lequel la feuille d'atomisation microporeuse (12) comprend une région microporeuse, et la section transversale de la chambre d'atomisation (14) et la section transversale de la région microporeuse sont circulaires :
et la chambre d'atomisation (14), la région microporeuse de la feuille d'atomisation microporeuse (12) et le tube à aiguille (13) sont disposés de manière coaxiale ;
**caractérisé en ce que** le diamètre de la chambre d'atomisation (14) est supérieur au diamètre de la région microporeuse et inférieur à deux fois le diamètre de la région microporeuse.

2. Le dispositif électronique d'atomisation selon la revendication 1, dans lequel le diamètre de la chambre d'atomisation (14) est compris entre 4 mm et 5 mm : et la distance entre l'extrémité du tube à aiguille (13) qui est située à proximité de la feuille d'atomisation microporeuse (12) et la paroi latérale de la chambre d'atomisation (14) est comprise entre 1,2 mm et 1,8 mm.

3. Le dispositif électronique d'atomisation selon la revendication 1, dans lequel la distance entre l'extrémité du tube à aiguille (13) située à proximité de la feuille d'atomisation microporeuse (12) et la feuille d'atomisation microporeuse (12) est comprise entre 0,2 mm et 0,4 mm.

4. Le dispositif électronique d'atomisation selon la revendication 1, dans lequel l'extrémité de la base d'atomisation (11) engagée avec la feuille d'atomisation microporeuse (12) est pourvue d'une rainure annulaire (114), et un élément d'étanchéité (115) est disposé dans la rainure annulaire (114) ; et une région non microporeuse de la feuille d'atomisation microporeuse (12) recouvre la rainure annulaire (114).

5. Le dispositif électronique d'atomisation selon la revendication 4, dans lequel la rainure annulaire (114) est annulaire, l'élément d'étanchéité est annulaire, et l'élément d'étanchéité (115) est fabriqué en silicone.

6. Le dispositif électronique d'atomisation selon la revendication 1, dans lequel l'extrémité de la base d'atomisation (11) engagée avec la feuille d'atomisation microporeuse (12) est pourvue d'un trou (113), la feuille d'atomisation microporeuse (12) couvre ce trou (113) et est engagée avec le trou (113) pour former la chambre d'atomisation (14), et l'extrémité du tube d'aiguille (13) proche de la feuille d'atomisation microporeuse (12) est éloignée par rapport à la paroi latérale du trou (113).

7. Le dispositif électronique d'atomisation selon la revendication 6, dans lequel le trou (113) est un trou traversant ou un trou borgne.

8. Le dispositif électronique d'atomisation selon la revendication 1, dans lequel l'extrémité du tube à aiguille (13) proche de la feuille d'atomisation microporeuse (12) est recouverte par une gaine de tube (131), et la gaine du tube est configuré pour augmenter l'adhérence du liquide à atomiser.

9. Le dispositif électronique d'atomisation selon la revendication 8, dans lequel la paroi extérieure de la gaine du tube (131) est éloignée de la paroi latérale de la chambre d'atomisation (14), et la gaine du tube (131) est fabriqué en silicone.

10. Le dispositif électronique d'atomisation selon la revendication 1, dans lequel le tube à aiguille (13) est un tube métallique cylindrique, et le diamètre intérieur du tube à aiguille (13) est compris entre 0,7 mm et 1,0 mm.

11. Le dispositif électronique d'atomisation selon la revendication 10, comprenant en outre un dispositif de commande (31), où un conducteur est disposé sur le tube à aiguille (13), et le conducteur est connecté électriquement au dispositif de commande (31) ; dans lequel la feuille d'atomisation microporeuse (12) est connectée électriquement au dispositif de commande (31), et le tube à aiguille (13) et la feuille d'atomisation microporeuse (12) forment un dispositif de détection de l'impédance ; et dans lequel le dispositif de commande (31) détecte la valeur de résistance entre le tube à aiguille (13) et la feuille de pulvérisation microporeuse (12) et contrôle l'état de fonctionnement de la feuille de pulvérisation microporeuse (12) en fonction de la valeur de résistance.

12. Le dispositif électronique d'atomisation selon la revendication **1,** dans lequel la portion d'extrémité de la base d'atomisation (11) éloignée de la feuille d'atomisation microporeuse (12) est pourvue d'une rainure de logement (116), où la rainure de logement est configurée pour accommoder le dispositif de stockage de liquide, et l'extrémité du tube à aiguille (13) éloignée de la feuille d'atomisation microporeuse (12) est disposée dans la rainure de logement (116).
